# DEMANDE DE BREVET EUROPEEN

(11) **EP 0 679 389 A1**
(43) Date de publication de la demande: **02.11.1995**
(21) Numéro de dépôt: 95400759.7
(22) Date de dépôt: 05.04.1995
(51) Int. Cl.: A61K 7/50, A61K 7/02

(54) **Composition cosmétique à base de composés quaternaires phosphates et de diesters de polyéthylèneglycol**

(30) Priorité: 27.04.1994 FR 9405087
(71) Demandeur: LA ROCHE POSAY Laboratoire Pharmaceutique, F-86270 La Roche Posay (FR)
(72) Inventeur: Brissonnet, Jean-Pierre, F-86210 Vouneuil Sur Vienne (FR); Burnier, Véronique, F-86100 Chatellerault (FR)
(74) Mandataire: Tezier Herman, Béatrice

(57) **Abrégé**

L'invention concerne une nouvelle composition cosmétique contenant un support constitué d'une phase grasse et d'une phase aqueuse, et qui comprend (i) un diester de polyéthylèneglycol et (ii) un composé quaternaire phosphaté de formule (I) : 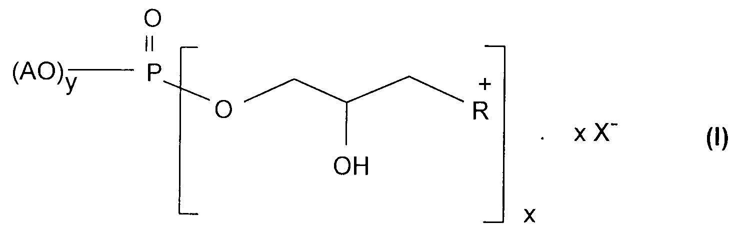
dans laquelle R est une amine tertiaire ayant au moins 6 atomes de carbone et qui peut être cyclique ou non, aliphatique, aromatique ou hétérocyclique ; X est un anion, de préférence un halogénure ; A est un métal alcalin ; x et y sont deux nombres entiers tels que (x+y) = 3, x ne pouvant être nul.
Elle concerne également l'utilisation de cette composition pour le démaquillage et/ou le nettoyage de la peau.

## Description

La présente invention se rapporte à une composition cosmétique contenant, dans un support constitué d'une phase grasse et d'une phase aqueuse, certains composés spécifiques, cette composition étant plus particulièrement destinée au démaquillage de la peau et/ou des yeux, ou seulement à leur nettoyage.

L'invention se rapporte également à un procédé de démaquillage qui consiste essentiellement à appliquer la composition conforme à l'invention sur de la peau et/ou sur des yeux maquillés.

Les démaquillants classiquement utilisés à ce jour sont des compositions ayant une concentration élevée en matières grasses dont la fonction, une fois appliquées sur la peau, est de dissoudre les différents corps gras qui sont contenus dans les produits de maquillage, de manière à les éliminer.

Ainsi, il est par exemple connu, par le document JP-A-55-150402, d'utiliser des compositions démaquillantes contenant des esters dont le nombre total d'atomes de carbone varie de 17 à 36 avec une préférence pour des esters ayant un nombre de carbone compris entre 23 et 28. La quantité efficace de ces esters est d'au moins 20% en poids par rapport au poids total de la composition.

Toutefois, l'utilisation de telles compositions démaquillantes à teneur élevée en corps gras provoque, au moment de leur application, un désagément ou inconfort qui se traduit par une sensation de lourdeur sur le visage ou de voile sur les yeux. L'application de ces compositions sur les yeux peut, par ailleurs, entraîner un gonflement des paupières.

La concentration élevée en corps gras pose également des problèmes d'odeur. Il est par conséquent nécessaire de masquer cette odeur par un parfumage intense, ce qui provoque d'autres problèmes de tolérance.

En outre, en raison de leur texture "lourde", ces démaquillants manquent de fraîcheur, sont difficiles à travailler, et leur rinçage n'est pas aisé.

En effet, après leur application sur la peau, telle que celle des paupières, un rinçage à l'aide d'un tonique ou d'eau s'avère indispensable.

Aussi, il subsiste dans l'état de l'art un fort besoin quant à pouvoir disposer d'une composition cosmétique constituée d'une phase grasse et d'une phase aqueuse, qui ne nécessite pas un rinçage obligatoire, qui ne mousse pas lors de l'application sur la peau, qui présente une faible concentration en corps gras tout en permettant d'obtenir un démaquillage correct de la peau.

La présente invention vise à la satisfaction d'un tel besoin.
Ainsi, à la suite d'importantes recherches menées sur la question, la demanderesse a maintenant découvert, de façon inattendue et surprenante, que l'utilisation d'une composition cosmétique, nouvelle en soi, constituée d'une phase grasse et d'une phase aqueuse, et comprenant au moins un diester de polyéthylèneglycol d'une part, et, d'autre part, au moins un composé quaternaire phosphaté spécifique de formule (I) suivante : 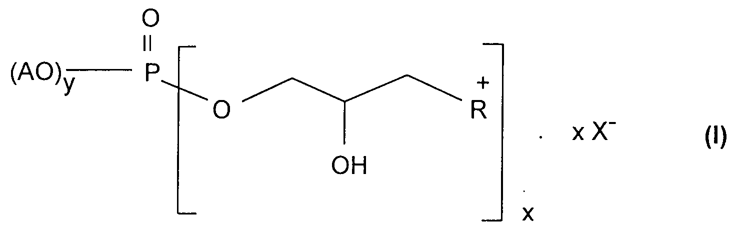
dans laquelle R est une amine tertiaire ayant au moins 6 atomes de carbone et qui peut être cyclique ou non cyclique, aliphatique, aromatique ou hétérocyclique, X est un anion, A est un métal alcalin et x et y sont deux nombres entiers tels que (x+y) = 3, x ne pouvant être nul,
permettait de nettoyer et/ou de démaquiller de manière efficace la peau et/ou les yeux sans qu'aucune irritation ou gène quelconque n'en résulte pour l'utilisateur.

Par ailleurs, la composition selon l'invention présente l'avantage de réaliser un démaquillage sans étape de rinçage obligatoire, ce qui est particulièrement intéressant dans le cas d'application sur une peau ayant certaines affections cutanées ou dans le cas d'application sur la peau dans des conditions peu propices à un rinçage de la peau, tel que dans le cas de voyage.

Un autre avantage de la composition selon l'invention est qu'elle est adaptée à l'élimination de tout type de produits de maquillage, y compris les maquillages aquarésistants ("waterproof") pour les yeux ou les produits de maquillage aux textures riches en corps gras tels que des fonds de teint, poudres, rouges-à-lèvres, servant notamment au maquillage des acteurs.

Enfin, un dernier avantage intéressant attaché à la composition selon l'invention réside dans le fait que celle-ci peut être employée dans les pays chauds, où l'utilisation de démaquillants trop riches en corps gras donne une sensation de lourdeur sur la peau, souvent mal supportée.

Mais d'autres caractéristiques, aspects, et avantages de l'invention apparaîtront encore plus clairement à la lecture de la description détaillée qui va suivre.

Selon l'invention, le radical R représente de préférence un motif amidoamine de formule (II) suivante : 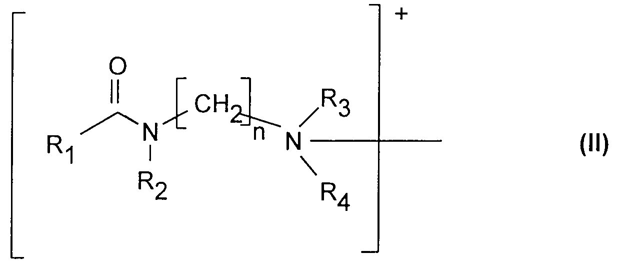
dans laquelle
- R₁ désigne un groupement choisi parmi un groupement alkyl, alcényl, alcoxy ou hydroxyalkyl ayant de 5 à 22 atomes de carbone chacun, ou un groupement aryl ou alkaryl ayant jusqu'à 20 atomes de carbone,
- R₂ est choisi parmi l'atome d'hydrogène, un groupement alkyl, hydroxyalkyl ou alcényl ayant jusqu'à 6 atomes de carbone chacun, un groupement cycloalkyl ayant jusqu'à 6 atomes de carbone, et de préférence de 2 à 5 atomes de carbone, ou un groupement polyoxyalkylène ayant jusqu'à 10 atomes de carbone,
- R₃ et R_{4,}, identiques ou différents, sont choisis parmi un groupement alkyl, hydroxyalkyl ou carboxyalkyl ayant jusqu'à 6 atomes de carbone dans chaque entité alkyl, ou un groupement polyoxyalkylène ayant jusqu'à 10 atomes de carbone, lesdits radicaux R₃ et R₄ pouvant par ailleurs former ensemble un N-hétérocycle avec l'atome d'azote auquel ils sont rattachés,
- et n est un entier allant de 2 à 10.

L'anion entrant dans la formule (I) est de préférence choisi parmi les halogénures, tels que les ions fluorure, chlorure, bromure.

Le métal alcalin utilisé est, de préférence, choisi parmi le sodium, le lithium et le potassium.

Selon un mode particulièrement préféré de réalisation de la présente invention, le composé de formule (I) est choisi parmi le chlorure de stéaramidopropyl PG-dimonium phosphate, le chlorure de linoléamidopropyl PG-dimonium phosphate, le chlorure de cocamidopropyl PG-dimonium phosphate.

Ce composé est généralement compris en une quantité allant de 0,5 à 5 % en poids, et de préférence en une quantité allant de 1 à 2 % en poids, par rapport au poids total de la composition.

Les diesters avantageusement utilisés pour la préparation de la composition selon l'invention sont ceux obtenus par réaction entre un acide gras, saturé ou non, ayant de 16 à 22 atomes de carbone, et un polyéthylèneglycol dont la répétition du motif oxyéthyléné va de 150 à 175.

Encore plus préférentiellement, les diesters utilisés dans la composition sont choisis parmi les polyéthylèneglycol distéarates, les polyéthylèneglycol dipalmitates, les polyéthylèneglycol dioléates, les polyéthylèneglycol dibéhénates.

Le diester entrant dans la composition est présent en une quantité allant généralement de 1 à 5 % en poids, et de préférence en une quantité allant de 1 à 2 % en poids, par rapport au poids total de la composition.

La composition selon l'invention comprend en outre au moins un corps gras, constitutif de la phase grasse, et qui est choisi de préférence parmi les alcools gras, les huiles ayant un point de fusion supérieur à 30 °C.

Plus précisément encore, les alcools gras peuvent être choisis parmi l'alcool cétylique, l'alcool stéarylique et leur mélange. Parmi les huiles à point de fusion supérieur à 30 °C, on peut plus particulièrement utiliser le beurre de karité, le beurre d'illipé, le beurre de cacao.

Selon une caractéristique particulièrement avantageuse de la présente invention, la phase aqueuse rentrant dans la composition conforme à l'invention, représente au moins 90% en poids, de préférence au moins 95% en poids, et enfin encore plus préférentiellement au moins 97% en poids, du poids total de la composition.

Cette phase aqueuse de la composition conforme à l'invention est constituée de préférence d'eau, dans laquelle se trouve contenu au moins un composé de formule générale (I) ci-dessus.

La composition peut éventuellement comprendre en outre au moins un parfum, et au moins un conservateur compris en une quantité allant de 0,1 à 1% en poids par rapport au poids total de la composition.

La composition selon l'invention peut se présenter sous forme d'une émulsion (eau-dans-huile, huile-dans-eau), d'une dispersion, d'un gel, d'une crème, d'une mousse ou sous toute autre forme généralement utilisées dans le domaine cosmétique.

L'invention se rapporte également à un procédé de démaquillage de la peau qui consiste à appliquer une composition telle que décrite ci-avant sur de la peau et/ou sur des yeux maquillés. Comme indiqué précédemment, l'application de cette composition sur la peau ne provoque pas l'apparition de mousse.

Ce procédé peut éventuellement comprendre une étape de rinçage, qui n'est pas obligatoire.

D'autre caractéristiques et avantages de la composition selon l'invention pourront apparaître dans l'exemple qui suit, qui est donné à titre purement illustratif et nullement limitatif.

### EXEMPLE

On a préparé ici une composition utilisable pour le démaquillage de la peau et/ou son nettoyage, et qui se présente sous la forme d'une suspension.

Les proportions pondérales sont données en pourcentage en poids par rapport au poids total de la composition.

| Phase grasse : | |
|---|---|
| Alcool cétylique (facteur de consistance) | 1 |
| Alcool stéarylique (facteur de consistance) | 1 |
| Beurre de karité (émolient) | 1,5 |
| Polyéthylèneglycol distéarate | 2 |

| Phase aqueuse : | |
|---|---|
| Cocamidopropyl PG-dimonium Chlorure phosphate | 1 |
| Conservateur | 0,1 |
| Eau qsp | 100 |

Cette composition a été obtenue en introduisant à 75°C la phase grasse dans la phase aqueuse, puis en agitant l'ensemble pour homogénéiser. Un parfum est finalement introduit en une quantité suffisante à une température inférieure à 50°C après homogénéisation des deux phases.

Cette composition permet un nettoyage et/ou démaquillage correct et non aggressif des paupières et des contours des yeux.

## Revendications

**1-** Composition cosmétique contenant un support constitué d'une phase grasse et d'une phase aqueuse, caractérisée par le fait qu'elle comprend (i) un diester de polyéthylèneglycol et (ii) un composé quaternaire phosphaté de formule (I) : 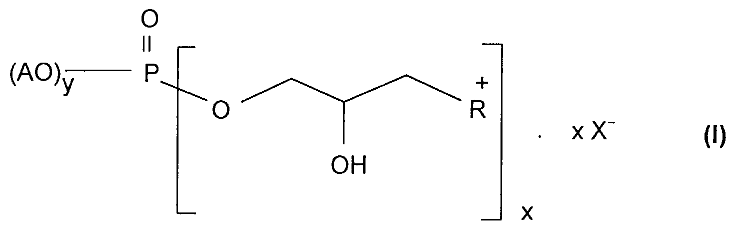 dans laquelle
- R est une amine tertiaire ayant au moins 6 atomes de carbone et qui peut être cyclique ou non cyclique, aliphatique, aromatique ou hétérocyclique,
- X est un anion,
- A est un métal alcalin,
- x et y sont deux nombres entiers tels que (x+y) = 3, x ne pouvant être nul.

**2-** Composition selon la revendication 1, caractérisée en ce que le radical R de la formule (I) est un motif amidoamine de formule (II) : 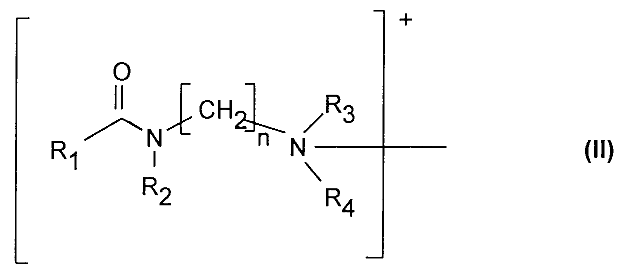 dans laquelle
- R₁ est choisi parmi un groupement alkyl, alcényl, alcoxy ou hydroxyalkyl ayant de 5 à 22 atomes de carbone chacun, ou un groupement aryl ou alkaryl ayant jusqu'à 20 atomes de carbone,
- R₂ est choisi parmi l'atome d'hydrogène, un groupement alkyl, hydroxyalkyl ou alcényl ayant jusqu'à 6 atomes de carbone chacun, un groupement cycloalkyl ayant jusqu'à 6 atomes de carbone, et de préférence de 2 à 5 atomes de carbone, ou un groupement polyoxyalkylène ayant jusqu'à 10 atomes de carbone,
- R₃ et R₄, identiques ou différents, sont choisis parmi un groupement alkyl, hydroxyalkyl ou carboxyalkyl ayant jusqu'à 6 atomes de carbone dans chaque entité alkyl, ou un groupement polyoxyalkylène ayant jusqu'à 10 atomes de carbone, lesdits radicaux R₃ et R₄ pouvant par ailleurs former ensemble un N-hétérocycle avec l'atome d'azote auquel ils sont rattachés,
- n est un entier allant de 2 à 10.

**3-** Composition selon l'une des revendications 1 ou 2, caractérisée en ce que l'anion est choisi parmi les halogénures, de préférence chlorure.

**4-** Composition selon l'une des revendications 1 à 3, caractérisée en ce que le composé de formule (I) est choisi parmi le chlorure de stéaramidopropyl PG-dimonium phosphate, le chlorure de linoléamidopropyl PG-dimonium phosphate, le chlorure de cocamidopropyl PG-dimoniumphosphate.

**5-** Composition selon l'une des revendications 1 à 4, caractérisée en ce que le diester de polyéthylèneglycol est choisi parmi les diesters d'acides gras et de polyéthylèneglycol dont l'acide gras a de 16 à 22 atomes de carbone et dont la chaine polyéthylèneglycol a de 150 à 175 motifs d'oxyde d'éthylène.

**6-** Composition selon l'une des revendications 1 à 5, caractérisée en ce que le diester de polyéthylèneglycol est choisi parmi les polyéthylèneglycol distéarates, les polyéthylèneglycol dipalmitates, les polyéthylèneglycol dioléates, les polyéthylèneglycol dibéhénates.

**7-** Composition selon l'une des revendications 1 à 6, caractérisée en ce qu'elle comprend au moins un corps gras choisi parmi les alcools gras, les huiles ayant un point de fusion supérieur à 30 °C.

**8-** Composition selon la revendication 7, caractérisée en ce que l'alcool gras est choisi parmi l'alcool cétylique, l'alcool stéarylique, l'alcool isohexadécylique, et leurs mélanges.

**9-** Composition selon la revendication 7, caractérisée en ce que les huiles à point de fusion supérieur à 30°C sont choisies parmi le beurre de karité, le beurre d'illipé, le beurre de cacao et leur mélange.

**10-** Composition selon l'une des revendications 1 à 9, caractérisée en ce que le composé de formule (I) est compris en une quantité allant de 0,5 à 5 % en poids par rapport au poids total de la composition.

**11-** Composition selon la revendication 10, caractérisée en ce que le composé de formule (I) est compris en une quantité allant de 1 à 2 % en poids par rapport au poids total de la composition.

**12-** Composition selon l'une des revendications 1 à 11, caractérisée en ce que le diester de polyéthylèneglycol est compris en une quantité allant de 1 à 5 % en poids par rapport au poids total de la composition.

**13-** Composition selon la revendication 12, caractérisée en ce que le diester de polyéthylèneglycol est compris en une quantité allant de 1 à 2 % en poids par rapport au poids total de la composition.

**14-** Composition selon l'une des revendications 1 à 13, caractérisée en ce que la quantité de phase aqueuse représente au moins 90 % en poids du poids total de la composition.

**15-** Composition selon la revendication 14, caractérisée en ce que ladite quantité est d'au moins de 95 % en poids.

**16-** Composition selon la revendication 15, caractérisée en ce que ladite quantité est d'au moins 97 % en poids.

**17-** Composition selon l'une des revendications précédentes, caractérisée en ce qu'il s'agit d'une composition non moussante pour le nettoyage et/ou le démaquillage.

**18-** Utilisation d'une composition telle que définie à l'une quelconque des revendications précédentes comme, ou pour la fabrication d'une, composition nettoyante et/ou démaquillante.

**19-** Procédé de démaquillage, caractérisé en ce que l'on applique sur de la peau et/ou sur des yeux maquillés une composition selon l'une quelconque des revendications 1 à 17.

**20-** Procédé de nettoyage, caractérisé en ce que l'on applique sur de la peau et/ou sur des yeux à nettoyer une composition selon l'une quelconque des revendications 1 à 17.

**21-** Procédé selon la revendication 19 ou 20, caractérisé en ce qu'il comprend éventuellement une étape complémentaire de rinçage.
